# EUROPEAN PATENT SPECIFICATION

(11) **EP 0 399 175 B1**
(45) Date of publication and mention of the grant of the patent: **03.09.1997**
(21) Application number: 90105854.5
(22) Date of filing: 28.03.1990
(51) Int. Cl.: A61K 31/557

(54) **PGE1 and derivatives for dermal or mucosal application**
PGE1 und Derivate für mukosale oder dermale Anwendung
PGE1 et dérivés à application mucosale ou dermique

(30) Priority: 04.04.1989 DE 3910760
(43) Date of publication of application: 28.11.1990
(73) Proprietor: Beiersdorf Aktiengesellschaft, D-20245 Hamburg (DE)
(72) Inventor: Melnik, B., Dr. Med., 4018 Langenfeld (DE); Plewig, G., Prof. Dr. med., 4000 Düsseldorf 30 (DE)
(74) Representative: Werner, Hans-Karsten, Dr.Dipl.-Chem.

(56) References cited:
- EP-A- 0 115 419
- EP-A- 0 391 218
- US-A- 4 009 282
- DIALOG INFORMATION SERVICES - FILE MEDLINE 155; A. SZCZEKLIK et al.: "Bronchial reactivity to prostaglandins F2alpha, E2, and histamine in different types of asthma"
- DIALOG INFORMATION SERVICES, FILE BIOSIS 5; M. SCHALIN-KARRILA et al.: "Evening primrose oil in the treatment of atopic eczema effect on clinical status plasma phospholipid fatty acids and circulating blood prostaglandins"
- DIALOG INFORMATION SERVICES, FILE BIOSIS 5; P. EBDEN et al.: "A study of evening primrose seed oil in atopic asthma"
- DIALOG EMBASE FILE 72, B. MELNIK et al.: "A new concept of the aetiopathogenesis and prevention of atopy"

## Description

The invention relates to the new use of Prostaglandin E₁, Prostaglandin E₁ derivatives and Prostaglandin E₁ analogs, the physiologically compatible salts, esters, amides, phospholipids and glycolipids thereof for the topical and inhalative treatments of acute manifest atopies in the dermal and mucosal areas and a combination with another active substance.

The Japanese Laid-Open Patent Application [JP-OS] 88/145230 (63/145230) relates to the use of Prostaglandin E₁ as a therapeutic and prophylactic pharmaceutic for the treatment of certain brain diseases. Furthermore, it has been known from the JP-OS 87/223120 (62/223120) that AIDS may possibly be controlled by a combination treatment with various prostaglandins. According to the EP-A-257 859, a combination tissue plasminogen activator-prostaglandin can be used as a thrombolytic agent.
The EP-A-100 630 relates to analogs of Prostaglandin E₁ which can be employed for protection against cytotoxic substances, above all in the liver. From the German Laid-Open Patent Application DE-OS 33 15 356 it has been known that Prostaglandin E₁ derivatives and their cyclodextrin inclusion complexes as well as their salts can be employed, when administered orally or parenterally, against brain ischemia. Various other literature references relate to the synthesis and use of PGE 1 derivatives and analogs.

So, US-A-4,009,282 teaches the treatment of proliferating skin diseases with prostaglandins.
There is, however, no drug on the market with PGE₁.

So far, however, for the treatment of atopy there have been generally used substances such as antihistamines, glucocorticoids and beta-sympatheticomimetic agents. Nevertheless, in practice since long there has been a great need for fast-acting substances which, especially in the case of an acute manifest atopy in the dermal and mucosal regions will provide an immediate relief.

M. Schalin-Karrila (in Br. J. Dermatol. 117 (1), 1987, 11-20) reported in DIALOG BIOSIS Previews File 5 (Title: Evening primrose oil in the treatment of atopic eczema effect on clinical status plasma phospholipid fatty acids and circulating blood prostaglandins) that primrose oil is active against atopic eczema.

EP-A-0,381,823 teaches a combination of cytotropic heterogenous molecular lipids (CHML) and a process for preparing the same. This can include prostaglandins, however, only Prostaglandin E₂ and not Prostaglandin E₁. The combination of CHML can injure carcinoma cells and various virus. It can enhance the immunity of the body. There is, however, no indication about an activity as topical or inhalative treatment of acute manifest atopies in the dermal and mucosal areas.

It is the object of the present invention to provide an immediately effective agent in cases of acute manifest atopies in the dermal and mucosal areas.

Said object can been attained by a surprisingly simple route by using one or more substances selected from,
PGE₁, PGE₁ derivatives and PGE₁ analogs, their physiologically compatible salts, esters, amides, phospholipids and glycolipids
and, in addition, one or more substances selected from
gamma-linolenic acid, 15-hydroxy-dihomo-gammalinolenic acid and dihomo-gamma-linolenic acid, the physiologically compatible salts, esters, amides, phospholipids and glycolipids thereof
for the manufacture of a medicament for the topical or inhalative treatment of acute manifest atopy in the dermal and mucosal areas.

A further object is a combination of one or more substances selected from
PGE₁, PGE₁ derivatives and PGE₁ analogs, their physiologically compatible salts, esters, amides, phospholipids and glycolipids
and, in addition, one or more substances selected from
gamma-linolenic acid, 15-hydroxy-dihomo-gammalinolenic acid and dihomo-gamma-linolenic acid, the physiologically compatible salts, esters, amides, phospholipids and glycolipids thereof
for the topical or inhalative treatment of acute manifest atopy in the dermal and mucosal areas.

The combination according to the invention can be used for the topical or inhalative application in the form of eye drops, nose sprays, creams, lotions or also in the form of aerosols.

Gamma-linolenic acid is present, *inter alia*, in the oil of the black evening primrose *(Oenothera biennis)* and in the seeds of black currants. In addition, there is a number of other natural sources of gamma-linolenic acids (e.g. *Borago officinalis)*. Dihomo-gamma-linolenic acid is obtainable, *inter alia*, by the process according to the EP-A-252 716 from *Mortierella* cultures.

Now, the advantage of the present invention above all is to be seen in that by virtue of the Prostaglandin E₁ therapy there is immediate relief from the atopic symptoms. Furthermore, the use of the substances according to the invention in combination with gamma-linolenic acid and/or dihomo-gamma-linolenic acids and/or the pharmacologically reasonable dosage forms thereof contributes to some prevention, since these substances, as a rule, are only slowly metabolized to form PGE₁.

## Claims

1. Use of one or more substances selected from
PGE₁, PGE₁ derivatives and PGE₁ analogs, their physiologically compatible salts, esters, amides, phospholipids and glycolipids
and, in addition, one or more substances selected from
gamma-linolenic acid, 15-hydroxy-dihomo-gammalinolenic acid and dihomo-gamma-linolenic acid, the physiologically compatible salts, esters, amides, phospholipids and glycolipids thereof
for the manufacture of a medicament for the topical or inhalative treatment of acute manifest atopy in the dermal and mucosal areas.

2. A combination of one or more substances selected from
PGE₁, PGE₁ derivatives and PGE₁ analogs, their physiologically compatible salts, esters, amides, phospholipids and glycolipids
and, in addition, one or more substances selected from
gamma-linolenic acid, 15-hydroxy-dihomo-gammalinolenic acid and dihomo-gamma-linolenic acid, the physiologically compatible salts, esters, amides, phospholipids and glycolipids thereof
for the topical or inhalative treatment of acute manifest atopy in the dermal and mucosal areas.

## Patentansprüche

1. Verwendung von einer oder mehreren Substanzen, die aus
PGE₁, PGE₁-Derivaten und PGE₁-Analoga, ihren physiologisch verträglichen Salzen, Estern, Amiden, Phospholipiden und Glycolipiden
ausgewählt sind, und außerdem von einer oder mehreren Substanzen, die aus
γ-Linolensäure, 15-Hydroxydihomo-γ-linolensäure und Dihomo-γ-linolensäure, den physiologisch verträglichen Salzen, Estern, Amiden, Phospholipiden und Glycolipiden davon
ausgewählt sind, zur Herstellung eines Medikaments für die topische oder inhalative Behandlung von akuter manifester Atopie in den Haut- und Schleimhautbereichen.

2. Kombination von einer oder mehreren Substanzen, die aus
PGE₁, PGE₁-Derivaten und PGE₁-Analoga, ihren physiologisch verträglichen Salzen, Estern, Amiden, Phospholipiden und Glycolipiden
ausgewählt sind, und außerdem von einer oder mehreren Substanzen, die aus
γ-Linolensäure, 15-Hydroxydihomo-γ-linolensäure und Dihomo-γ-linolensäure, den physiologisch verträglichen Salzen, Estern, Amiden, Phospholipiden und Glycolipiden davon
ausgewählt sind, für die topische oder inhalative Behandlung von akuter manifester Atopie in den Haut- und Schleimhautbereichen.

## Revendications

1. Utilisation d'une ou plusieurs substances choisies parmi
PGE₁, les dérivés de PGE₁ et les analoques de PGE₁, les sels, esters, amides, phospholipides et glycolipides physiologiquement compatibles de ceux-ci,
et en plus d'une ou plusieurs substances choisies parmi
l'acide γ-linolénique, l'acide 15-hydroxydihomo-γ-linolénique, l'acide dihomo-γ-linolénique, les sels, esters, amides, phospholipides et glycolipides physiologiquement compatibles de ceux-ci,
pour la préparation d'un médicament pour le traitement topique ou inhalateur de l'atopie aiguë manifeste dans les régions dermiques ou mucodermiques.

2. Combination d'une ou plusieurs substances choisies parmi
PGE₁, les dérivés de PGE₁ et les analogues de PGE₁, les sels, esters, amides, phospholipides et glycolipides physiologiquement compatibles de ceux-ci,
et en plus d'une ou plusieurs substances choisies parmi
l'acide γ-linolénique, l'acide 15-hydroxydihomo-γ-linolénique, l'acide dihomo-γ-linolénique, les sels, esters, amides, phospholipides et glycolipides physiologiquement compatibles de ceux-ci,
pour le traitement topique ou inhalateur de l'atopie aiguë manifeste dans les régions dermiques ou mucodermiques.
